# EUROPEAN PATENT APPLICATION

(11) **EP 4 534 084 A1**
(43) Date of publication of application: **09.04.2025**
(21) Application number: 23815146.8
(22) Date of filing: 29.05.2023
(51) Int. Cl.: A61K 31/4375, A61P 17/00, A61K 8/40, A61Q 19/00, A61Q 19/02

(54) **USE OF TETRAHYDRONAPHTHYRIDINE DERIVATIVE FOR PREPARING PRODUCT FOR IMPROVING HYPERPIGMENTATION**

(30) Priority: 30.05.2022 CN 202210597120
(71) Applicant: Scinnohub Pharmaceutical Co., Ltd., Chengdu, Sichuan 610094 (CN)
(72) Inventor: HU, Xiao, Chengdu, Sichuan 610094 (CN); WANG, Yan, Chengdu, Sichuan 610094 (CN); JI, Sen, Chengdu, Sichuan 610094 (CN); WANG, Xiao, Chengdu, Sichuan 610094 (CN); ZHANG, Xiaodong, Chengdu, Sichuan 610094 (CN); TANG, Jun, Chengdu, Sichuan 610094 (CN)
(74) Representative: Gille Hrabal Partnerschaftsgesellschaft mbB Patentanwälte
(86) International application number: PCT/CN2023/096844
(87) International publication number: WO 2023/231966

(57) **Abstract**

Provided are use of a compound represented by formula I, a pharmaceutically acceptable salt, a hydrate, an isomer, a prodrug, or a mixture thereof for preparing a product for improving hyperpigmentation. The compound has a good application prospect for treating melasma.

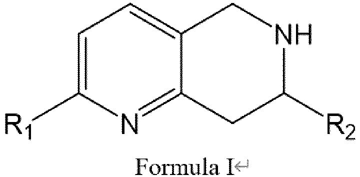

## Description

### CROSS-REFERENCE TO RELATED APPLICATION

This application claims the priority to the prior Application No. 202210597120.9 filed to China National Intellectual Property Administration on May 30, 2022 and entitled "Use of tetrahydronaphthyridine derivative for preparing product for improving hyperpigmentation", the entire contents of which are incorporated herein by reference.

### TECHNICAL FIELD

The present disclosure relates to the field of chemistry, particularly to use of a tetrahydronaphthyridine derivative for preparing a product for improving hyperpigmentation, and more particularly to use of a tetrahydronaphthyridine derivative for preparing a product for improving melasma.

### BACKGROUND

Skin hyperpigmentation is a difficult disease in dermatology, such as melasma, age spots, freckles, melanosis, facial moles, and mongolian spots, which not only affects appearance but also seriously affects people's life experiences. With the increasing improvement of people's living standards, whitening, removing spots and improving skin hyperpigmentation have gradually become people's common pursuits and it is also the needs in the treatment of many dermatological diseases.

Melasma is a very common chronic acquired skin disease with increased facial melanin, mainly characterized by increased pigmentation. Melasma, also called liver spots and butterfly spots, is more common in middle-aged women. Its main symptoms are dark brown or yellowish-brown patches or spots with blurry borders that are symmetrically distributed on the cheeks, forehead, and mandible. Patients with melasma usually have no subjective symptoms and do not need treatment, but melasma may seriously affect the appearance and cause physical and psychological damage to the patients. With the improvement of living standards, people pay more and more attention to their appearance, and the treatment of skin diseases such as melasma has also received more and more attention.

Presently, the pathogenesis of melasma is still not completely identified. Many studies have shown that genetic susceptibility, ultraviolet radiation, endocrine dysfunction, skin barrier dysfunction, changes in hormone levels, mental factors, and nutritional levels are closely related to the occurrence of melasma. In the actual treatment process, due to the different sizes and severity of melasma and varying degrees of pigmentation, melasma lesions of patients are usually classified and categorized clinically to develop a comprehensive therapeutic regimen. Presently, the methods of drug treatment for melasma include topical and systemic medication.

Topical medication is usually based on the following two aspects: the first aspect is to protect or restore the skin's barrier function by using functional skin care products, and the other aspect is to inhibit and eliminate melanin in melasma lesions by using topical drugs mainly for epidermal melasma. The representative drug is hydroquinone, which may inhibit the synthesis of melanocyte DNA and RNA, competitively bind to tyrosinase, inhibit the formation of melanosomes, or accelerate their degradation. As the drug concentration increases, the decolorization effect is stronger, but the irritation to the skin is stronger as well, and a burning sensation may occur, and occasionally local allergic reactions may occur. The common concentration of hydroquinone is 2%-5%. In 2002, the FDA approved triple combination cream (TCC) consisting of 4% hydroquinone, 0.01% fluocinolone and 0.05% retinoic acid for the treatment of melasma, which is currently the first-line drug for the treatment of melasma. However, due to its many adverse reactions, the TCC should not be used more than half a year.

The most representative drug for systemic medication is tranexamic acid (TXA). TXA is a plasmin inhibitor, including oral, topical application, and local microneedle injection. In recent years, it has shown good effect in the treatment of melasma, and has been proven to improve melasma. TXA achieves the effect of fading spots by inhibiting plasmin, reducing α-melanocyte stimulating hormone (α-MSH), reducing melanin production, and further competitively inhibiting tyrosinase activity in melanosomes. In addition, TXA can inhibit vascular proliferation and reduce erythema; some studies have further suggested that TXA can improve melasma, which is related to its ability to inhibit the expression of endothelin-1 (ET-1). There are some side effects of TXA when used for melasma. Some literature has reported that oral administration of TXA causes headaches and severe abdominal distension, and patients give up treatment because they cannot bear it. The local use of TXA causes adverse reactions such as erythema and a burning sensation.

Although there are currently a variety of options available for improving melasma, their effects are not particularly satisfactory; besides, the medication cycle is very long and the frequency of medication is high, making it difficult to meet the increasing clinical needs. The development of safer and more effective products for hyperpigmentation is still the focus of concern in the current stage.

### SUMMARY

An object of the present disclosure is to provide use of a compound represented by formula I, a pharmaceutically acceptable salt, a hydrate, an isomer, a prodrug, or a mixture thereof for preparing a product for improving hyperpigmentation, 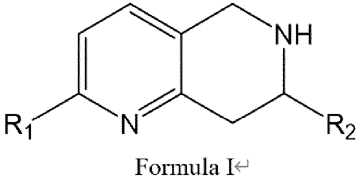
where, R₁ is selected from carboxyl, a phosphate group, and a sulfonic acid group;
R₂ is selected from hydrogen, substituted or unsubstituted amino, substituted or unsubstituted C₁-C₆ alkyl, substituted or unsubstituted C₁-C₆ alkoxyl, C₁-C₄ halogenated alkyl, substituted or unsubstituted C₃-C₆ cycloalkyl, substituted or unsubstituted 4- to 8-membered aliphatic heterocyclic group, substituted or unsubstituted 6- to 10-membered aryl, and a substituted or unsubstituted 6- to 10-membered aromatic heterocyclic group.

In some specific embodiments, R₂ is selected from hydrogen, and the following groups unsubstituted or optionally substituted by one, two or more R₂ₐ: amino, C₁-C₆ alkyl, C₁-C₆ alkoxyl, C₁-C₄ halogenated alkyl, C₃-C₆ cycloalkyl, 4- to 8-membered aliphatic heterocyclic group, 6- to 10-membered aryl, and a 6- to 10-membered aromatic heterocyclic group.

In some specific embodiments, the R₂ₐ is selected from halogen, OH, NH₂, NO₂, CN, oxo (=O), C₁-C₆ alkyl, C₁-C₆ alkoxyl, C₃-C₆ cycloalkyl, 4- to 8-membered aliphatic heterocyclic group, 6- to 10-membered aryl, and a 6- to 10-membered aromatic heterocyclic group.

In some specific embodiments, the R₂ is selected from hydrogen, phenyl unsubstituted or optionally substituted by one, two or more R₂ₐ, and phenyl-C₁-C₆ alkyl.

In some specific embodiments, the R₂ is selected from hydrogen and

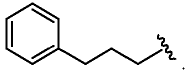

In some specific embodiments, the hyperpigmentation can be a hyperpigmentation-related disease, and the hyperpigmentation-related disease is a disease caused by the deposition of melanin on the skin surface, including melasma, age spots, freckles, melanosis, facial moles, and mongolian spots, etc.

In some specific embodiments, the hyperpigmentation-related disease is melasma.

In some specific embodiments, the compound represented by formula I is selected from a compound with the following chemical structure, a pharmaceutically acceptable salt, a hydrate, an isomer, a prodrug, or a mixture thereof:

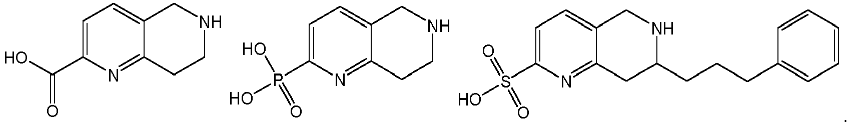

In some specific embodiments, the pharmaceutically acceptable salt is hydrochloride of the compound represented by formula I.

In some specific embodiments, the product for improving hyperpigmentation can be a drug or a cosmetic.

In some specific embodiments, the product for improving hyperpigmentation is a pharmaceutical preparation for clinical use prepared by at least one of a compound represented by formula I, a pharmaceutically acceptable salt, a hydrate, an isomer, a prodrug thereof as an active ingredient, and one or more pharmaceutically acceptable excipients. The pharmaceutical preparation of the present disclosure can be prepared into an oral preparation, an external preparation, an injection, and the like for use.

### Beneficial effects

In the present disclosure, rat models of melasma are induced by UVB irradiation at 280-320 nm and injection of progesterone, and drugs are administered by local injection. The effect of the compound is evaluated by apparent indexes, histopathological examination, biochemical indexes, etc. The results show that the compound represented by formula I of the present disclosure can effectively reduce the number of melanin granules in the skin surface cells of the modeling area of melasma model rats, improve the skin tissue structure morphology and inflammatory cell infiltration, and effectively reduce the levels of melasma-related biochemical indexes, which has a good application prospect for the treatment of melasma.

### BRIEF DESCRIPTION OF DRAWINGS

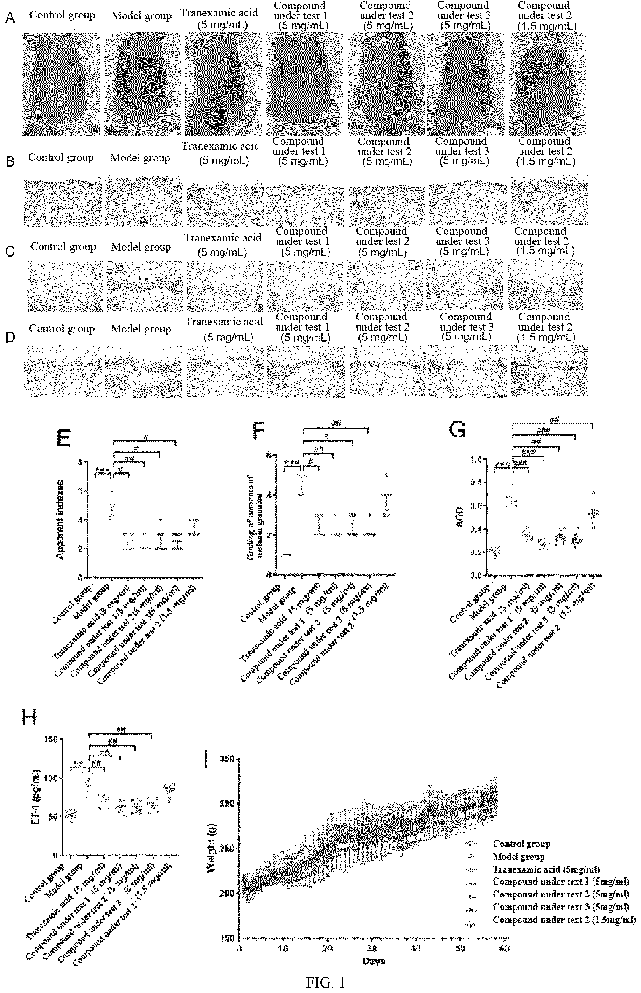

FIG. 1 shows the effects of tranexamic acid and groups of compounds under test on rat models of melasma induced by ultraviolet irradiation and injection of progesterone. (A) overall condition of rat skin in the experimental area; (B) HE staining of the skin tissues in each group; (C) distribution of melanin granules in the epidermal basal layer and stratum spinosum in each group; (D) expression of tyrosinase in the skin tissues in each group; (E) observation and grading of the apparent indexes of the melasma models; (F) grading of the content of melanin granules in the rat models of melasma after collection of skin tissues on Day 58; (G) distribution of the average integrated optical density of epidermal tyrosinase in each group after collection of skin tissues on Day 58; (H) content of ET-1 in the supernatant of the skin tissue homogenate of rats in each group after collection on Day 58; (I) changes in body weights of the rats in each group during the experiment period (Day 1 to Day 58). All scoring and counting results are obtained by a double-blind method. ***P<0.001 compared with the control group, # P <0.05, ## P < 0.01, ### P < 0.001 compared with the model group.

### Definitions and explanations of terms

Unless otherwise specified, the definitions of groups and terms recorded in the specification and claims of this application, including their definitions as examples, exemplary definitions, preferred definitions, definitions recorded in tables, definitions of specific compounds in embodiments, etc., can be arbitrarily combined. The definitions of groups and compound structures after such combinations should be construed to be within the scope recorded in the specification and/or claims of this application.

Unless otherwise specified, the numerical range recorded in this specification and claims is equivalent to recording at least each specific integer value therein. For example, the numerical range "1-40" is equivalent to recording every integer value in the numerical range "1-10", i.e., 1, 2, 3, 4, 5, 6, 7, 8, 9, 10, and every integer value in the numerical range "11-40", i.e., 11, 12, 13, 14, 15, 16, 17, 18, 19, 20, 21, 22, 23, 24, 25, 26, 27, 28, 29, 30, 31, 32, 33, 34, 35, 36, 37, 38, 39, 40. Furthermore, when certain numerical ranges are defined as "numbers", it should be understood that the two endpoints of the range, every integer in the range, and every decimal in the range are recorded. For example, "a number from 0 to 10" should be understood as not only recording each integer of 0, 1, 2, 3, 4, 5, 6, 7, 8, 9, and 10, but also recording at least the sum of each of these integers with 0.1, 0.2, 0.3, 0.4, 0.5, 0.6, 0.7, 0.8, and 0.9 respectively.

It should be understood that in the description of 1, 2, or more, "more" should refer to an integer greater than 2, for example, greater than or equal to 3, for example, 3, 4, 5, 6, 7, 8, 9 or 10.

The term "halogen" represents fluorine, chlorine, bromine, and iodine.

"C₁-C₆ alkyl" represents straight and branched alkyl having 1, 2, 3, 4, 5 or 6 carbon atoms. The alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, and the like or an isomer thereof.

The term "alkoxyl" refers to -O-(alkyl), where alkyl is as defined above. Non-limiting examples of alkoxyl include methoxyl, ethoxyl, propoxyl, and butoxyl.

The term "C₃-C₆ cycloalkyl" should be understood as a saturated monovalent monocyclic hydrocarbon ring having 3, 4, 5 or 6 carbon atoms. The C₃₋₁₀ cycloalkyl may be monocyclic hydrocarbyl, for example, cyclopropyl, cyclobutyl, cyclopentyl, and cyclohexyl.

Unless otherwise defined, the term "4- to 8-membered aliphatic heterocyclic group" refers to a saturated ring or ring system, for example, a 4-, 5-, 6- or 7-membered monocyclic ring, a 7- or 8-bicyclic ring (such as a fused ring, a bridged ring, a spiro ring), and contains at least one, for example, 1, 2, 3, 4, 5 or more heteroatoms selected from O, S and N, where N and S may also be optionally oxidized to various oxidation states to form states of nitrogen oxides, - S(O)- or -S(O)₂-. The aliphatic heterocyclic group may be connected to the rest of the molecule via any of the carbon atoms or a nitrogen atom (if any). The aliphatic heterocyclic group may include a fused or bridged ring and a spirocyclic ring. Particularly, the aliphatic heterocyclic group may include, but is not limited to, a 4-membered ring, for example, azetidinyl and oxetanyl; a 5-membered ring, for example, tetrahydrofuranyl, dioxolyl, pyrrolidinyl, imidazolidinyl, pyrazolidinyl, and pyrrolinyl; or a 6-membered ring, for example, tetrahydropyranyl, piperidinyl, morpholinyl, dithianyl, thiomorpholinyl, piperazinyl or trithianyl; or a 7-membered ring, for example, diazepanyl.

The term "6- to 10-membered aryl" should be preferably understood as a monovalent aromatic or partially aromatic monocyclic or bicyclic ring having 6, 7, 8, 9 or 10 carbon atoms, particularly a ring having 6 carbon atoms ("C₆ aryl"), for example, phenyl; or biphenyl, or a ring having 9 carbon atoms ("C9 aryl"), for example, indanyl or indenyl, or a ring having 10 carbon atoms ("C10 aryl"), for example, tetrahydronaphthyl, dihydronaphthyl or naphthyl. When the 6- to 10-membered aryl is substituted, it may be monosubstituted or polysubstituted. Furthermore, there is no limitation on the substitution site, for example, it may be ortho-, para- or meta-substituted.

The term "6- to 10-membered aromatic heterocyclic group" should be understood as including such a monovalent monocyclic or bicyclic (e.g., fused ring, bridged ring, spiro ring) aromatic ring system having 6 to 10 ring atoms and containing 1 to 5 heteroatoms independently selected from N, O and S, preferably containing 1 to 3 heteroatoms independently selected from N, O and S. "Heteroaryl" also refers to a group in which a heteroaromatic ring is fused to one or more aromatic, alicyclic or heterocyclic rings, where the radical or point of attachment is on the heteroaromatic ring. For example, it is selected from pyridyl, pyrimidinyl, pyrazinyl, pyridazinyl, and pyranyl.

### DETAILED DESCRIPTION

The technical solutions of the present disclosure will be further described in detail below in conjunction with specific embodiments. It should be understood that the following examples are only used for exemplary illustrations and explanations of the present disclosure and should not be construed as limiting the scope of protection of the present disclosure. All technologies implemented based on the above content of the present disclosure are included in the scope of protection intended by the present disclosure.

Unless otherwise specified, the raw materials and reagents used in the following examples are commercially available products or can be prepared by known methods.

### 1. Experimental purpose

The experimental purpose is to test the therapeutic effect of the compound of the present disclosure on rat melasma models.

### 2. Experimental materials and instruments

### Experimental reagents

| Reagent | Article No. | Manufacturer |
|---|---|---|
| Masson-Fontana melanin staining solution | G2032 | Beijing Solarbio Science & Technology Co., Ltd. |
| Progesterone injection | NMPAApproval No. H33020828 | Zhejiang Xianju Pharmaceutical Co., Ltd. |
| Tranexamic acid | PHR1812 | Sigma |
| Sterile water for injection | NMPAApproval No. H33022485 | Zhejiang Shapuaisi Pharmaceutical Co., Ltd. |
| 1 M sodium hydroxide aqueous solution | S0542 | Tokyo Chemical Industry |
| Tyrosinase antibody | AF5491 | Affinity |
| Endothelin 1 ELISA Kit | Ab133030 | Abcam |
| 10% neutral formaldehyde | G2161 | Beijing Solarbio Science & Technology Co., Ltd. |
| Monohydrate citric acid | 108902129 | Shanghai Lingfeng Chemical Reagent Co., Ltd. |
| Sodium bicarbonate | 10018960 | Sinopharm Chemical Reagent Co., Ltd. |
| HE staining solution | KGA224 | Jiangsu KeyGEN Biotech Corp., Ltd |
| Ready-to-use immunohistochemistry kit | KIT-9720 | Fuzhou MXB Biotechnologies Development Co., Ltd. |
| Anhydrous ethanol | 10009257 | Sinopharm Chemical Reagent Co., Ltd. |
| Hydrogen peroxide | 88597 | Sigma |
| Xylene | 10023418 | Sinopharm Chemical Reagent Co., Ltd. |

### Experimental instruments

| Name | Model | Manufacturer |
|---|---|---|
| Microscope | Nikon Eclipse E200 | Nanjing Nikon Jiangnan Optical Instrument Co., Ltd. |
| Oven | DHG-9053B5-III | Shanghai CIMO Medical Instrument Co., Ltd. |
| Paraffin slicer | RM2016 | Shanghai Leica Instrument Co., Ltd. |
| Slide drier | KD-P | Zhejiang Jinhua Kedee Instrument Equipment Co., Ltd. |
| Oven | GFL-230 | Tianjin Laiborui Instrument Equipment Co., Ltd. |
| Embedding machine | JB-P5 | Wuhan Junjie Electronics Co., Ltd. |
| Freezing table | JB-L5 | Wuhan Junjie Electronics Co., Ltd. |
| Electronic balance | ME155DU/02 | Mettler Toledo |
| Berthold LB941microplate | Berthold LB941 | Berthold |
| multifunctional microplate reader | | |
| UV lamp | NB-UVB311 | Philips |
| Electronic scale | X2 | Kaifeng Group Co., Ltd. |

### 3. Experimental method

3.1 Modeling: Before the experiment, on Day 0, SD rats (purchased from Beijing Vital River Experimental Animal Technology Co., Ltd., SPF grade, female, 7-8 weeks old, weighing 200±20 g) were randomly grouped and numbered. The hairs on the backs of animals in each group were shaved using an electric shaver, and the short hairs on the skin were removed with a depilatory cream to fully expose the back skin. The hair removal area was slightly larger than the actual modeling area (5 cm×5 cm). Each rat was depilated once every 2-3 days to ensure that the back skin was fully exposed. On the next day (Day 1), rats in each group were weighed, and the weights were recorded when the electronic scale reading was stable. A progesterone injection was injected intramuscularly at the base of the rat's thigh (alternately between the thighs on both sides), with a drug concentration of 20 mg/mL and an injection dose of 25 mg/kg, once a day. After the intramuscular injection, the rat's back skin was irradiated with ultraviolet light at a wavelength of 280-320 nm. The light source was about 30 cm away from the rat's back skin, and the irradiation time lasted for 60 minutes each time, for a total of 4 weeks.

3.2 Administration: After modeling, positive control tranexamic acid (5 mg/mL) and an injection of a compound under test (5 mg/mL and 1.5 mg/mL, with normal saline as the solvent) were injected to multiple sites on the skin lesions of the rats using a 30 G insulin needle. The injection volume was 0.05 mL/cm², 5 days each time, and the administration lasted for 30 days continuously. The animals in the control group (only depilated, without modeling) and the model group were given the solvent.

3.3 Detection of indicators: The first day of modeling was denoted as Day 1, and the experimental period lasted for 58 days. After the end of the experiment (Day 58), 1 hour after the last administration, the skin of the modeling area of the rats in each group was photographed for scoring of apparent indexes (scoring criteria was provided in Table 1). Then the rats in each group were euthanized, their backs were depilated, and 0.5 g of full-thickness skin tissue from the depilated back was taken and placed into an ice bath, and normal saline was added to make a 10% tissue homogenate. The homogenate was centrifuged at 3000 r/min at room temperature for 10 min to obtain a supernatant of skin tissue homogenate. ELISA was carried out to detect the expression of endothelin-1 in the supernatant of skin tissue homogenate. The skin of the modeling area on the back of rats was taken and immersed in 10% neutral formaldehyde for fixation at room temperature for 4 days. Four days later (Day 62), paraffin embedding and sectioning were performed to obtain paraffin sections of skin. The HE staining was performed to observe the pathological changes of the skin tissues of rats in each group. Staining method: The dried paraffin sections were conventionally dewaxed with xylene, hydrated with descending gradient ethanol, and washed with distilled water; the nuclei were stained with hematoxylin for 2 minutes, differentiated with hydrochloric acid and alcohol for a few seconds, and washed with water to return to blue; the sections were stained with eosin stain for 1 minute, and the residual stain was washed with water; the sections were dehydrated and dried with gradient alcohol, transparentized with xylene, and blocked with neutral gum, then observed under microscope. The epidermal hyperplasia of the skin tissue sample sections, arrangement of basal cells, presence or absence of inflammatory cell infiltration in the dermis, and changes in the number of capillaries in all groups were observed according to the evaluation indexes. Fontana-Masson staining was performed to observe the skin pigmentation of the rats. The experimental procedures were as follows: after dewaxing and hydration, the sections were immersed in a Fontana silver nitrate solution and incubated in a 56°C incubator in the dark for 30-40 min, and washed with distilled water for 5-6 times, 1-2 minutes each time; then the sections were treated with a sodium thiosulfate solution for 1-5 min, and rinsed with tap water for 3-5 min; then counterstained with a neutral red staining solution for 5 minutes, and rinsed with tap water for 1 minute; dehydrated with 95% ethanol and anhydrous ethanol, transparentized with xylene, and blocked with neutral gum, then observed under microscope. The distribution of melanin granules was scored (scoring criteria were provided in Table 2). An immunohistochemistrical assay was performed to detect the expression of tyrosinase in the skin in each group. The experimental procedures were as follows: 1) drying of sections: the prepared paraffin sections were placed in an electric constant temperature drying oven and dried at 60°C for 3 hours; 2) the dried paraffin sections were dewaxed conventionally with xylene, hydrated with descending gradient ethanol, and washed with distilled water; 3) antigen retrieval; 4) 3% H₂O₂ was added dropwise and incubated at room temperature for 10 minutes to inactivate endogenous enzymes, and then washed with PBS three times, 3 minutes each time; 5) Normal goat immune serum was added dropwise to each section for blocking, after incubation for 10 minutes at room temperature, the excess liquid was shaken off. Washing was not allowed. Then tyrosinase antibody diluted in a certain proportion (1:50) was added dropwise and stored in a refrigerator at 4°C overnight; 6) After being taken out of the refrigerator and restored to room temperature, the sections were washed with a PBS three times, 3 minutes each time; 7) A polymer enhancer (reagent A) was added dropwise to each section, and kept at room temperature for 20 minutes, and then washed with the PBS three times, 3 minutes each time; 8) An enzyme-labeled anti-mouse/rabbit polymer (reagent B) was added dropwise to each section, and kept at room temperature for 10 minutes, and then washed with the PBS three times, 3 minutes each time; 9) DAB was added for color development, and the reaction time was control under microscope, then hematoxylin counterstaining was performed, and distilled water was added for washing to stop color development; 10) The sections were dehydrated and dried with gradient alcohol, transparentized with xylene, and blocked with neutral gum; 11) The sections were photographed under a phase contrast microscope at 200× magnification. The area of tyrosinase-positive area (stained area) and the integrated optical density were measured by the software ImageJ (1.8.0). The average optical density was calculated by the formula: AOD=IOD/Area.

**Table 1 Grading Criteria of Apparent Indexes of Melasma**

| Grade | Lesion Color | Lesion Area (cm²) |
|---|---|---|
| 0 | Normal skin | None |
| 1 | Light brown | <2 |
| 2 | Brown | 2-4 |
| 3 | Dark brown | >4 |

| | | |
|---|---|---|
| Total score=area score+color score | | |

**Table 2 Scoring Criteria for Local Pathological Observation by Fontana-Masson Staining**

| Score | Distribution of melanin granules |
|---|---|
| 1 | Melanin occasionally observed in the basal layer and stratum spinosum of the epidermis |
| 2 | Melanin distributed discontinuously, mainly in the basal layer |
| 3 | Band-shaped melanin granules distributed continuously, mainly in the base layer and stratum spinosum |
| 4 | Band-shaped melanin granules distributed continuously and mainly in the base layer and stratum spinosum, and more melanin caps occurring in the keratinocytes |
| 5 | Melanin granules distributed densely in the epidermis and more melanin caps existing |

3.4 The experimental data were represented by means ± SD. The one-way ANOVA and Tukey's test were utilized to analyze the statistical differences among groups. The Mann-Whitney U test was utilized to analyze the scoring data; P<0.05 was considered significantly different.

### 3.5 Preparation of compounds under test

### Preparative example 1

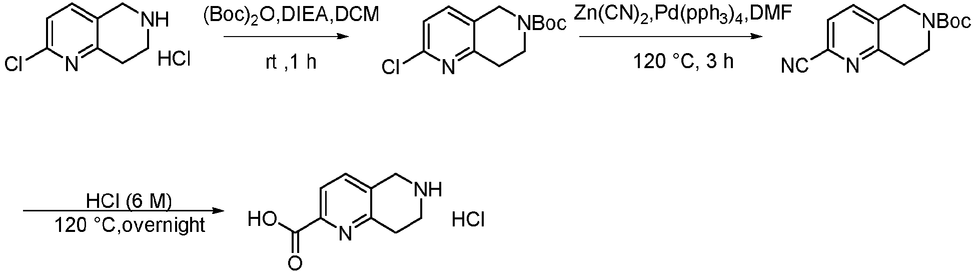

### Step 1: Preparation of tert-butyl 2-chloro-7,8-dihydro-1,6-naphthyridine-6-(5H)-carboxylate

2-chloro-5,6,7,8-tetrahydro-1,6-naphthyridine hydrochloride (0.9 g) was weighed and suspended in dichloromethane (15 mL), N,N-diisopropylethylamine (1.4 g) was added to dissociate, then di-tert-butyl dicarbonate (1.15 g) was added to react at room temperature for 1 hour. When the TLC showed the raw materials were completely consumed, purification was performed by column chromatography to obtain the title compound (1.12 g).

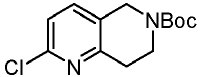

MS (ESI) m/z (M+H)⁺=269.0.

### Step 2: Preparation of tert-butyl 2-cyano-7,8-dihydro-1,6-naphthyridine-6-(5H)-carboxylate

Tert-butyl 2-chloro-7,8-dihydro-1,6-naphthyridine-6(5H)-carboxylate (1.12 g) was weighed and dissolved in N,N-dimethylformamide (20 mL), then zinc cyanide (2.44 g) and tetrakis (triphenylphosphine)palladium (483 mg) were added, and argon replacement was performed 3 times, and a reaction was carried out at 120°C for 3 hours. When the TLC showed the raw materials were completely consumed, ethyl acetate was added for dilution, and a reaction mixture was filtered with diatomaceous earth and extracted with ethyl acetate twice, washed with saturated brine once and dried over anhydrous sodium sulfate, and subjected to purification by column chromatography to obtain the title compound (1.1 g).

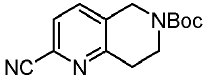

MS (ESI) m/z (M+H)⁺ =260.0.

### Step 3: Preparation of 5,6,7,8-tetrahydro-1,6-naphthyridine-2-carboxylic acid hydrochloride

Tertiary butyl 2-cyano-7,8-dihydro-1,6-naphthyridine-6-(SH)-carboxylate (1.1 g) was weighed and dissolved in a 6 M aqueous hydrochloric acid solution (25 mL), and reacted at 120 °C overnight. When the LCMS showed that the raw materials were completely consumed, a reaction solution was concentrated to dryness, and separated by pre-HPLC to obtain 5,6,7,8-tetrahydro-1,6-naphthyridine-2-carboxylic acid hydrochloride (compound under test 1, 726 mg).

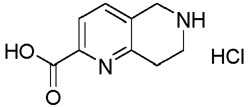

MS (ESI) m/z (M+H)⁺ =179.0.

¹H NMR (400 MHz, Methanol-d₄) δ 8.21 (d, *J =* 8.1 Hz, 1H), 8.12 (d, *J =* 8.0 Hz, 1H), 4.60 (s, 2H), 3.71 (t, *J =* 6.4 Hz, 2H), 3.42 (t, *J =* 6.4 Hz, 2H).

### Preparative example 2

### Step 1: Preparation of tert-butyl 2-chloro-7,8-dihydro-1,6-naphthyridine-6-(5H)-carboxylate

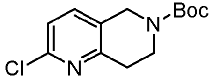

2-chloro-5,6,7,8-tetrahydro-1,6-naphthyridine hydrochloride (0.9 g) was suspended in dichloromethane (15 mL), and *N,N-* diisopropylethylamine (1.4 g) was added, subsequently di-tert-butyl dicarbonate (1.15 g) was added to obtain a mixture, and the mixture was reacted at room temperature for 1 hour. When the TLC showed that the raw materials were completely consumed, a reaction solution was diluted with water, and extracted with dichloromethane; organic phases were combined, dried over anhydrous sodium sulfate, filtered, and concentrated, and the obtained crude product was purified by column chromatography to obtain the target compound (1.12 g).

MS (ESI) m/z (M+H)⁺= 269.0.

### Step 2: Preparation of tert-butyl 2-(diethoxyphosphoryl)-7,8-dihydro-1,6-naphthyridine-6(5H)-carboxylate

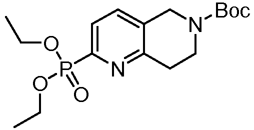

In an argon atmosphere, tert-butyl 2-chloro-7,8-dihydro-1,6-naphthyridine-6(5H)-carboxylate (100 mg) was dissolved in toluene (20 mL), and diethyl phosphate (102 mg), tris(dibenzylideneacetone)dipalladium (34 mg), 1,1'-bis(diphenylphosphino)ferrocene (41 mg) and triethylamine (75 mg) were added, and the system was reacted at 120 °C overnight. When the TLC showed that the raw materials were completely consumed, ethyl acetate was added to dilute, filtered with diatomaceous earth, a filtrate was collected and concentrated, and the obtained crude product was purified by preparative TLC to obtain the target compound (70 mg).

MS (ESI) m/z (M+H)⁺= 371.1.

### Step 3: Preparation of (5,6,7,8-tetrahydro-1,6-naphthyridin-2-yl)phosphonic acid hydrochloride (compound under test 2)

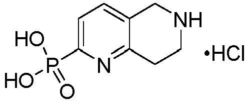

Tert-butyl 2-(diethoxyphosphoryl)-7,8-dihydro-1,6-naphthyridine-6(5*H*)-carboxylate (70 mg) was dissolved in concentrated hydrochloric acid (5 mL), and reacted overnight at 100 °C. When the LCMS showed that the raw materials were completely consumed, a reaction solution was concentrated, and the obtained crude product was purified by preparative HPLC to obtain the target compound (30 mg).

MS (ESI) m/z (M+H)⁺= 215.0.

¹H NMR (400 MHz, D₂O) δ 8.35 (dd, *J =* 8.0, 2.4 Hz, 1H), 8.06 (t, *J =* 7.7 Hz, 1H), 4.59 (s, 2H), 3.67 (t, *J =* 6.0 Hz, 2H), 3.49 (t, *J =* 6.4 Hz, 2H).

### Preparative example 3

### (1) Preparation of 2-methyl-N-(4-phenylbutylene)propane-2-sulfinamide

4-phenylbutanol (7.6 mL) was dissolved in dichloromethane (200 mL), then 2-iodoxybenzoic acid (28.00 g), tert-butanesulfinyl amide (9.07 g), anhydrous magnesium sulfate (30.80 g), and pyridinium toluene-4-sulphonate (0.627 g) were added in sequence to obtain a mixture. The mixture was heated to 40°C, reacted for 24 hours, then cooled to room temperature, and subjected to suction filtration to obtain a filter cake, and the filter cake was washed with dichloromethane, and a filtrate was concentrated to dryness and subjected to purification by column chromatography to obtain the title compound (5.1 g).

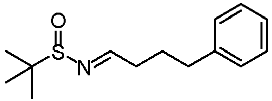

MS (ESI) m/z (M+H)⁺=252.1.

### (2) Preparation of N-(1-(3-bromo-6-methoxypyridin-2-yl)-5-phenylpentane-2-yl)-2-methylpropane-2-sulfinamie

3-bromo-6-methoxy-2-methylpyridine (4.85 g) was weighed and placed in a dry reaction bottle, then anhydrous tetrahydrofuran (100 mL) was injected in a nitrogen atmosphere to obtain a mixture, and the mixture was cooled to -78 °C; a tetrahydrofuran solution (12.00 mL, 2.0 M) of lithium diisopropylamide was added dropwise to react at -78 °C for 40 minutes. Then tetrahydrofuran (20 mL) containing 2-methyl-N-(4-phenylbutylidene)propane-2-sulfinamide (5.02 g) was added dropwise to react at -30 °C for 30 minutes to obtain a mixture, and the mixture was slowly heated to room temperature. When the LCMS showed that the reaction was completed, a saturated ammonium chloride solution was added to quench, ethyl acetate and water were added to obtain a mixture, the mixture was separated and extracted, and organic phase was concentrated to dryness; purification was performed by column chromatography to obtain the title compound (7.15 g).

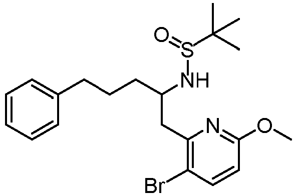

MS (ESI) m/z (M+H)⁺=453.1, 455.1.

### (3) Preparation of ethyl 2-(2-((tert-butylsulfinyl)amino)-5-phenylpentyl)-6-methoxynicotinate

N-(1-(3-bromo-6-methoxypyridin-2-yl)-5-phenylpentane-2-yl)-2-methylpropane-2-sulfinamide (5.42 g) was weighed and dissolved in ethanol (150 mL), then a [1,1'-bis(diphenylphosphino)ferrocene]dichloropalladium dichloromethane complex (0.979 g) and N,N-diisopropylethylamine (4.12 mL) were added; after the addition, the system was ventilated by carbon monoxide, reacted under a reflux condition overnight in a carbon monoxide atmosphere, and desolventized under reduced pressure; purification was performed by column chromatography to obtain the title compound (2.01 g).

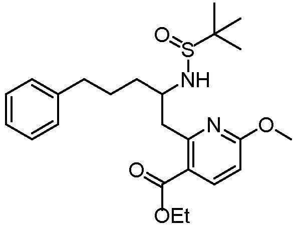

MS (ESI) m/z (M+H)⁺ =447.2.

### (4) Preparation of 2-methoxy-7-(3-phenylpropyl)-7,8-dihydro-1,6-naphthyridin-5(6H)-one

Ethyl 2-(2-((tert-butylsulfinyl)amino)-5-phenylpentyl)-6-methoxynicotinate (2.00 g) was weighed and dissolved in acetonitrile (50 mL), and cesium carbonate (5.87 g) was added to obtain a mixture; the mixture was heated to 80 °C and stirred overnight. When the LC-MS monitored that the reaction was completed, the obtained mixture was cooled to room temperature, and subjected to suction filtration to obtain a filter cake, and the filter cake was washed with dichloromethane, and a filtrate was concentrated to dryness; purification was performed by column chromatography to obtain the title compound (1.1 g).

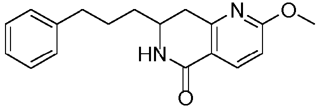

MS (ESI) m/z (M+H)⁺=297.1.

### (5) Preparation of 2-methoxy-7-(3-phenylpropyl)-5,6,7,8-tetrahydro-1,6-naphthyridine

2-methoxy-7-(3-phenylpropyl)-7,8-dihydro-1,6-naphthyridine-5(6H)-one (1.10 g) was weighed and dissolved in tetrahydrofuran (70 mL), and lithium aluminum hydride (0.562 g) was added in an ice bath, stirred at 70 °C for 8 hours, and monitored by LC-MS until the reaction was completed. In the ice bath, water (0.56 mL), a sodium hydroxide solution (15%, 0.56 mL), and water (1.68 mL) were added dropwise in sequence to obtain a mixture; after the addition, the mixture was stirred at room temperature for 20 minutes, dried with anhydrous magnesium sulfate, and subjected to suction filtration to obtain a filter cake, and the filter cake was washed with dichloromethane, and concentrated to dryness under reduced pressure; purification was performed by column chromatography to obtain the title compound (0.41 g).

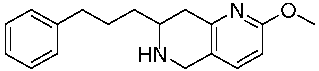

MS (ESI) m/z (M+H)⁺ =283.2.

### (6) Preparation of 7-(3-phenylpropyl)-5,6,7,8-tetrahydro-1,6-naphthyridine-2-ol

2-methoxy-7-(3-phenylpropyl)-5,6,7,8-tetrahydro-1,6-naphthyridine (0.40 g) was weighed, an acetic acid solution (5 mL) of hydrobromic acid was added to obtain a mixture, the mixture was heated to 80 °C and stirred for 5 hours, desolventized under reduced pressure, then ethyl acetate was added to pulp, and the mixture was filtered and dried to obtain the title compound (0.295 g).

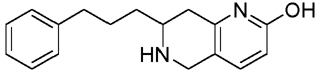

MS (ESI) m/z (M+H)⁺ =269.1.

### (7) Preparation of 2-chloro-7-(3-phenylpropyl)-5,6,7,8-tetrahydro-1,6-naphthyridine

7-(3-phenylpropyl)-5,6,7,8-tetrahydro-1,6-naphthyridine-2-ol (0.29 g) was weighed, phosphorus oxychloride (6 mL) was added to obtain a mixture, the mixture was heated to 100 °C and stirred for 10 hours, desolventized under reduced pressure, then ice water and dichloromethane were added, and a Na₂CO₃ aqueous solution was added to adjust the pH to 9-10, and the mixture was separated and extracted, and an organic phase was concentrated to dryness to obtain a crude product of the title compound (0.6 g).

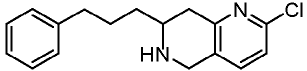

MS (ESI) m/z (M+H)⁺ =287.1.

### (8) Preparation of tert-butyl 2-chloro-7-(3-phenylpropyl)-7,8-dihydro-1,6-naphthyridine-6-(5H)-carboxylate

2-chloro-7-(4-methoxyphenylethyl)-5,6,7,8-tetrahydro-1,6-naphthyridine (0.6 g) was weighed and dissolved in dichloromethane (5 mL) and water (5 mL), a sodium carbonate solution was added to adjust the pH to 8-9, and then di-tert-butyl dicarbonate (0.50 mL) was added to obtain a mixture, the mixture was stirred at room temperature for 1 hour, concentrated to dryness under reduced pressure, and subjected to purification by column chromatography to obtain the title compound (0.22 g).

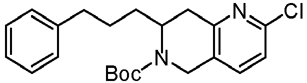

MS (ESI) m/z (M+H)⁺ =387.1.

### (9) Preparation of tert-butyl 2-(benzylthio)-7-(3-phenylpropyl)-7,8-dihydro-1,6-naphthyridine-6-(5H)-carboxylate

Tert-butyl 2-chloro-7-(3-phenylpropyl)-7,8-dihydro-1,6-naphthyridine-6-(5H)-carboxylate (147 mg), benzyl mercaptan (71 mg), tris(dibenzylideneacetone)dipalladium (35 mg), 4,5-bis(diphenylphosphine)-9,9-dimethylxanthene (44 mg), and triethylamine (113 uL) were weighed and added into a reaction flask, then 1,4-dioxane (5 mL) was added to obtain a mixture, the mixture was replaced with nitrogen and reacted at 100 °C overnight, and the mixture was concentrated to dryness under reduced pressure. Purification was performed by column chromatography to obtain the title compound (159 mg).

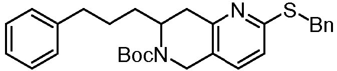

MS (ESI) m/z (M+H)⁺ =475.2.

### (10) Preparation of tert-butyl 2-(chlorosulfonyl)-7-(3-phenylpropyl)-7,8-dihydro-1,6-naphthyridine-6(5H)-carboxylate

Tert-butyl 2-(benzylthio)-7-(3-phenylpropyl)-7,8-dihydro-1,6-naphthyridine-6(5H)-carboxylate (156 mg) was weighed and dissolved in acetonitrile (5 mL), then acetic acid (79 mg), water (48 mg), and 1,3-dichloro-5,5-dimethylhydantoin (130 mg) were added in sequence in an ice bath to obtain a mixture. The mixture was stirred in the ice bath for 1 hour, then ice water and dichloromethane were added, and the mixture was separated and extracted. An organic phase was concentrated to dryness to obtain a crude product of the title compound (148 mg).

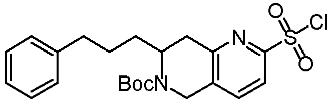

MS (ESI) m/z (M+H)⁺ =451.1.

### (11) Preparation of sodium 6-(tert-butyloxycarbonyl)-7-(3-phenylpropyl)-5,6,7,8-tetrahydro-1,6-naphthyridine-2-sulfonae

Tert-butyl 2-(chlorosulfonyl)-7-(3-phenylpropyl)-7,8-dihydro-1,6-naphthyridine-6(5H)-carboxylate (148 mg) was weighed and dissolved in tetrahydrofuran (5 mL) and water (5 mL), then sodium hydroxide (27 mg) was added to obtain a mixture, and the mixture was stirred at 80 °C for 1 hour. Ethyl acetate and water were added and the mixture was separated and extracted. An organic phase was discarded and an aqueous phase was concentrated to dryness to obtain a crude product of the title compound (50 mg).

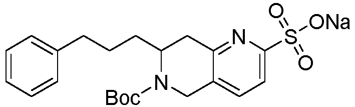

MS (ESI) m/z (M+H)⁺ =433.1.

### (12) Preparation of 7-(3-phenylpropyl)-5,6,7,8-tetrahydro-1,6-naphthyridine-2-sulfonic acid hydrochloride

The crude product of sodium (tert-butyloxycarbonyl)-7-(3-phenylpropyl)-5,6,7,8-tetrahydro-1,6-naphthyridine-2-sulfonate (50 mg) was weighed and dissolved in a 6 M hydrochloric acid solution (3 mL) to obtain a mixture, and the mixture was stirred at room temperature for 1 hour. The system was concentrated to dryness and separated by pre-HPLC to obtain the title compound (compound under test 3, 2.25 mg).

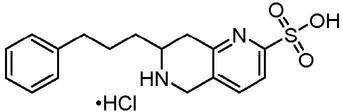

MS (ESI) m/z (M+H)⁺ =333.0.

¹H NMR (400 MHz, D₂O )δ 7.74 (dd, *J* = 18.5, 8.1 Hz, 2H), 7.24 (ddd, *J* = 21.9, 14.9, 7.3 Hz, 5H), 4.42 (s, 2H), 3.72-3.62 (m, 1H), 3.32-3.22 (m, 1H), 3.01-2.92 (m, 1H), 2.63 (d, *J* = 3.6 Hz, 2H), 1.83-1.58 (m, 4H).

### 4. Experimental results

The pharmacological activity of the compounds under test of the present disclosure is verified by the above experimental method. The statistical analysis of apparent, biochemical, and pathological indexes shows that the compounds under test of the present disclosure may effectively reduce skin hyperpigmentation of the rats and significantly improve the melasma symptoms of the model rats.

According to the experimental methods described in 3.1-3.4, on Day 58 (the day when the experiment ends), the collected experimental data is analyzed. The specific results are shown in FIG. 1.

4.1 The evaluation of the apparent indexes in FIGS. 1A and 1E show that there is no significant change in the back skin of the rats in the control group; there are obvious dark brown lesions and a larger lesion area in the rats in the model group; compared with the model group, the color of the lesion areas of the rats in the 5 mg/mL tranexamic acid and groups of the compounds under test is significantly lighter, showing light brown, and the lesion area is significantly reduced (P<0.05).

4.2 The HE staining results in FIG. 1B show that, in the control group, the epidermal tissue structure of the rats is intact, the stratum corneum cells are neatly arranged, there is no obvious fibroplasias and no inflammatory cell infiltration; compared with the control group, the epidermal tissues of the rats show obvious thickening of the granular layer, stratum spinosum and stratum corneum of the skin tissues, disordered skin tissue arrangement structure, uneven distribution, and inflammatory cell infiltration is observed in the model group. Compared with the model group, the thickness of the basal layer and stratum spinosum of the epidermal tissues is significantly reduced, the skin tissue structure arrangement gradually becomes flat, and the phenomenon of inflammatory cell infiltration is improved in the 5 mg/mL tranexamic acid and groups of the compounds under test.

4.3 The Fontana-Masson staining results in FIG. 1C show that the melanin granules are expressed positively and are in black color, and the nuclei are in red color; in the control group, there is almost no black positive area in the stained sections; in the model group, the black positive area in the epidermal sections is the largest, and the color is the darkest and is distributed in a continuous band shape, and the melanin granules are significantly increased, a large number of band-shaped melanin granules are distributed continuously in the basal cells and stratum spinosum, and densely distributed melanin granules and melanin caps appeare in the epidermis; FIG. 1F show the grading and evaluation of melanin granule content in skin tissues of the rats. Compared with the model group, the black positive area in the sections is reduced and the color becomes lighter, the melanin granules in the skin tissue are reduced, and the melanin granules are distributed in a band shape but mainly concentrated in the basal layer and stratum spinosum in the 5 mg/mL tranexamic acid group. Compared with the model group, the black positive area in the sections is reduced significantly and the color becomes significantly lighter, the melanin granules in the skin tissue are significantly reduced, and the melanin granules are distributed discontinuously only in the basal layer in the groups of compounds under test 1 and 3.

4.4 The immunohistochemical staining results of tyrosinase in FIG. 1D and FIG. 1G show that tyrosinase is positively expressed and is yellow-brown and the cell nuclei are blue. In the control group, there is almost no yellow-brown area in the immunohistochemical sections, indicating that the expression of tyrosinase in the rat skin tissues is low. In the model group, the tyrosinase positive area in the skin tissue sections is the largest, and the yellow-brown color is obvious, indicating that the tyrosinase positive rate in the skin tissues of the rats is high in the model group. Compared with the model group, the area of the tyrosinase positive expression areas in the skin tissue sections are significantly reduced and the color is significantly lighter in the group of the compound under test 1 and the group of the compound under test 3, indicating that the tyrosinase positive rate in the skin tissues is significantly reduced (P<0.001). The results in the groups of the compound under test 2 (1.5 mg/mL and 5 mg/mL) are significantly different from those in the model group (P<0.01).

4.5 FIG. 1H shows the detection of ET-1 content in the skin tissue homogenate by the ELISA method. The ET-1 content in the skin tissues of rats in the model group is significantly increased compared with that in the control group, and the ET-1 contents in the 5 mg/mL tranexamic acid and groups of the compounds under test are significantly reduced compared with the model group, showing significant differences therebetween (P<0.01).

4.6 FIG. 1I show that there is no significant difference in body weights of the rats among different groups during the entire experiment, indicating that the animals bear the compounds under test well.

The foregoing description describes the embodiments of the technical solutions of the present disclosure exemplarily. It should be appreciated that the protection scope of the present disclosure is not limited to the above embodiments. Any modification, equivalent substitution and improvement, etc. made by those skilled in the art within the spirit and principle of the present disclosure shall fall within the scope of protection as claimed by the appended claims of this application.

## Claims

1. Use of a compound represented by formula I, a pharmaceutically acceptable salt, a hydrate, an isomer, a prodrug, or a mixture thereof for preparing a product for improving hyperpigmentation, 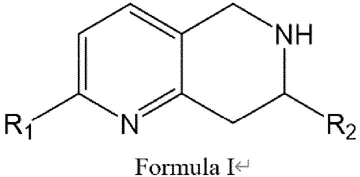 wherein, R₁ is selected from carboxyl, a phosphate group, and a sulfonic acid group; R₂ is selected from hydrogen, substituted or unsubstituted amino, substituted or unsubstituted C₁-C₆ alkyl, substituted or unsubstituted C₁-C₆ alkoxyl, C₁-C₄ halogenated alkyl, substituted or unsubstituted C₃-C₆ cycloalkyl, substituted or unsubstituted 4- to 8-membered aliphatic heterocyclic group, substituted or unsubstituted 6- to 10-membered aryl, and a substituted or unsubstituted 6- to 10-membered aromatic heterocyclic group.

2. The use according to claim 1, wherein R₂ is selected from hydrogen, and the following groups unsubstituted or optionally substituted by one, two or more R₂ₐ: amino, C₁-C₆ alkyl, C₁-C₆ alkoxyl, C₁-C₄ halogenated alkyl, C₃-C₆ cycloalkyl, 4- to 8-membered aliphatic heterocyclic group, 6- to 10-membered aryl, and a 6- to 10-membered aromatic heterocyclic group;
the R₂ₐ is selected from halogen, OH, NH₂, NO₂, CN, oxo (=O), C₁-C₆ alkyl, C₁-C₆ alkoxyl, C₃-C₆ cycloalkyl, 4- to 8-membered aliphatic heterocyclic group, 6- to 10-membered aryl, and a 6- to 10-membered aromatic heterocyclic group.

3. The use according to claim 1 or 2, wherein the R₂ is selected from hydrogen, phenyl unsubstituted or optionally substituted by one, two, or more R₂ₐ, and phenyl-C₁-C₆ alkyl.

4. The use according to any one of claims 1 to 3, wherein the R₂ is selected from hydrogen and

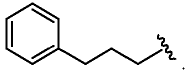

5. The use according to any one of claims 1 to 4, wherein the hyperpigmentation can be a hyperpigmentation-related disease, and the hyperpigmentation-related disease is a disease caused by the deposition of melanin on a skin surface.

6. The use according to claim 5, wherein the hyperpigmentation-related disease comprises one or more of melasma, age spots, freckles, melanosis, facial moles, and mongolian spots.

7. The use according to claim 5, wherein the hyperpigmentation-related disease is melasma.

8. The use according to any one of claims 1 to 7, wherein the compound represented by formula I is selected from a compound with the following chemical structure, a pharmaceutically acceptable salt, a hydrate, an isomer, a prodrug, or a mixture thereof:

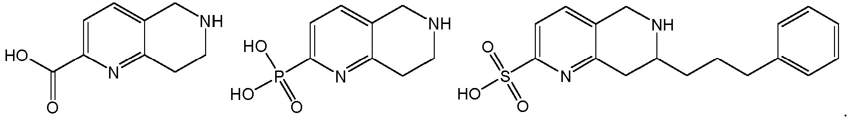

9. The use according to any one of claims 1 to 8, wherein the pharmaceutically acceptable salt is hydrochloride of the compound represented by formula I.

10. The use according to any one of claims 1 to 9, wherein the product for improving hyperpigmentation can be a drug or a cosmetic.
